# EUROPEAN PATENT APPLICATION

(11) **EP 4 303 294 A1**
(43) Date of publication of application: **10.01.2024**
(21) Application number: 22763293.2
(22) Date of filing: 02.03.2022
(51) Int. Cl.: C12M 1/00, C12N 5/071, C12N 5/073, C12N 5/078, C12N 5/0783

(54) **SAMPLING DEVICE AND CELL CULTIVATION SYSTEM**

(30) Priority: 03.03.2021 JP 2021033639
(71) Applicant: TERUMO Kabushiki Kaisha, Tokyo 151-0072 (JP)
(72) Inventor: IGARASHI, Masatsugu, Ashigarakami-gun, Kanagawa 259-0151 (JP)
(74) Representative: TBK
(86) International application number: PCT/JP2022/008732
(87) International publication number: WO 2022/186239

(57) **Abstract**

A cell culturing system (10) (sampling device (60)) includes a sampling channel (64), a detection unit (75), and a first measuring instrument (110) that measures a gas component contained in a sample by means of the detection unit (75) while the sample flows. The sampling device (60) also includes a standard solution storage unit (72) capable of supplying a standard solution to the detection unit (75) through the sampling channel (64), and a gas supply device (150) that supplies a gas having a predetermined component amount to the standard solution in the standard solution storage unit (72). The first measuring instrument (110) performs calibration by measuring the standard solution mixed with the gas having the predetermined component amount.

## Description

### Technical Field

The present invention relates to a sampling device for collecting a liquid sample from a culturing device that cultures cells, and a cell culturing system.

### Background Art

For example, U.S. Patent No. 9442047 discloses a sampling device including a sampling channel for collecting a liquid sample from a culturing device. The sampling device includes a pump that draws a sample from a sample introduction channel connected to the culturing device into the sampling channel, and a detection unit provided on a downstream side of the sampling channel. The detection unit detects components such as oxygen (O₂) or carbon dioxide (CO₂) in the sample and the amounts (concentrations) of the components.

### Summary of Invention

Meanwhile, a measuring instrument that performs measurement for the detection unit that detects O₂ and CO₂ needs to be calibrated at an appropriate timing. For example, the calibration of the measuring instrument is performed in such a way that a reference measurement value of the measuring instrument is set by the measuring instrument measuring a standard solution having a prescribed O₂ concentration and CO₂ concentration. To this end, the sampling device is equipped with a calibration device for bubbling (mixing) gas having a prescribed O₂ concentration and CO₂ concentration into the standard solution.

However, such a calibration device leads to an increase in size of the sampling device. In addition, a user needs to set the measuring instrument in the calibration device for calibration, which increases a work burden of the user.

The present invention has been made in view of the above circumstances, and an object thereof is to provide a sampling device and a cell culturing system capable of simply calibrating a measuring instrument with a burden of a user being significantly reduced.

In order to achieve the above object, a first aspect of the present invention provides a sampling device for collecting a sample in a liquid form from a culturing device that cultures a cell, the sampling device including: a sampling channel through which the sample flows; a detection unit provided in the sampling channel so as to come into contact with the sample; and a measuring instrument that measures at least a gas component contained in the sample by means of the detection unit while the sample flows, the sampling device including a standard solution storage unit capable of supplying a standard solution to the detection unit through the sampling channel, and a gas supply unit that supplies a gas having a predetermined component amount to the standard solution in the standard solution storage unit, wherein the measuring instrument performs calibration by measuring the standard solution mixed with the gas having the predetermined component amount.

In order to achieve the above object, a second aspect of the present invention provides a cell culturing system including a culturing unit for culturing a cell, the cell culturing system including: a sampling channel through which a sample in a liquid form collected from the culturing unit flows; a detection unit provided in the sampling channel so as to come into contact with the sample; and a measuring instrument that measures at least a gas component contained in the sample by means of the detection unit while the sample flows, the cell culturing system including a standard solution storage unit capable of supplying a standard solution to the detection unit through the sampling channel, and a gas supply unit that supplies a gas having a predetermined component amount to the standard solution in the standard solution storage unit, wherein the measuring instrument performs calibration by measuring the standard solution mixed with the gas having the predetermined component amount.

The sampling device and the cell culturing system enables easy calibration of the measuring instrument, and further, can achieve reduction in size and reduction in a burden of a user.

### Brief Description of Drawings

Fig. 1 is a perspective view schematically illustrating an overall configuration of a cell culturing system to which a sampling device according to an embodiment of the present invention is applied.
Fig. 2 is an explanatory diagram schematically illustrating a channel for a culture medium during cell culture.
Fig. 3 is an explanatory diagram schematically illustrating a channel of the sampling device.
Fig. 4 is a perspective view illustrating a first sensor unit and a second sensor unit.
Fig. 5 is an explanatory diagram schematically illustrating a standard solution storage unit and a gas supply device.
Fig. 6A is an explanatory diagram illustrating a gas supply device according to a first modification. Fig. 6B is an explanatory diagram illustrating a gas supply device according to a second modification.
Fig. 7 is a flowchart illustrating a sampling method performed by the sampling device.
Fig. 8 is an explanatory diagram illustrating operations in a priming step and a cleaning step.
Fig. 9 is an explanatory diagram illustrating an operation in a sampling step.
Fig. 10 is a flowchart illustrating a flowchart of processing in a calibration step.
Fig. 11A is an explanatory diagram illustrating an operation of supplying a first gas. Fig. 11B is an explanatory diagram illustrating a stand-by state in which the operation of the gas supply device is stopped. Fig. 11C is an explanatory diagram illustrating an operation of supplying a second gas.
Fig. 12 is an explanatory diagram illustrating an operation in a calibration step.
Fig. 13 is an explanatory diagram illustrating a gas supply device according to a third modification.

### Description of Embodiments

Preferred embodiments of the present invention will now be described in detail with reference to the drawings.

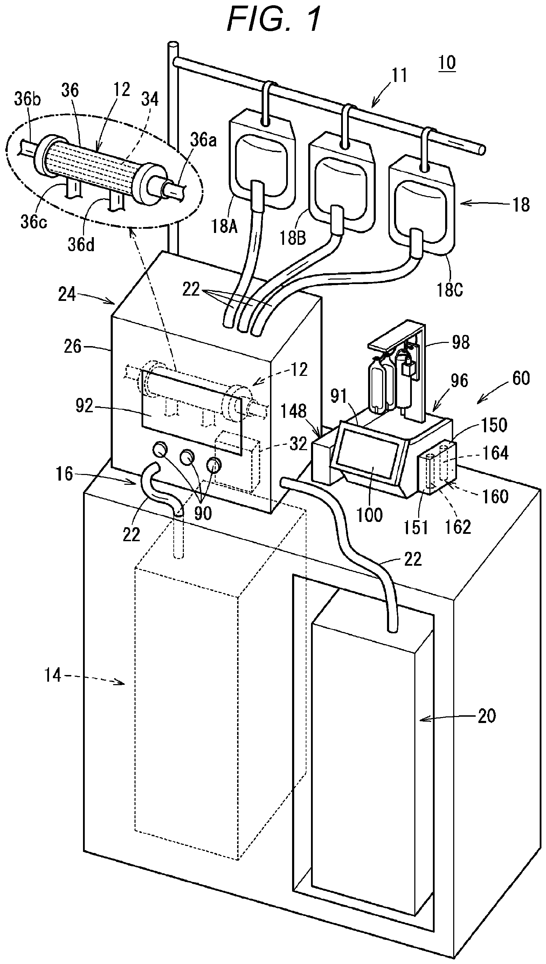

A sampling device 60 according to an embodiment of the present invention is applied to a cell culturing system 10 for culturing biological cells in regenerative medicine as illustrated in Fig. 1. The sampling device 60 samples a culture medium during culture of the cells by the cell culturing system 10, and measures the state of the culture medium. For example, the cell culturing system 10 continues cell culture for a long period of time by discharging lactic acid, carbon dioxide, or the like (including unused medium and oxygen) generated during cell culture from a reactor 12 which is a cell culture vessel while supplying a culture medium or oxygen to the reactor 12.

The biological cells are not particularly limited, and examples thereof include cells contained in blood (T cells and the like) and stem cells (ES cells, iPS cells, mesenchymal stem cells, etc.). As the culture medium, an appropriate culture medium may be selected according to biological cells, and examples thereof include those prepared by adding various amino acids, vitamins, serum, and the like to a basic solution that is, for example, a balanced salt solution (BSS).

The cell culturing system 10 includes a culturing device 11 (culturing unit) in which a reactor 12 is set and cells are actually cultured, and a sampling device 60 (sampling unit) that collects a liquid sample from the culturing device 11 during culture. Fig. 1 illustrates the culturing device 11 having only one reactor 12, but the culturing device 11 may include a plurality of reactors 12. In addition, the cell culturing system 10 may have a configuration in which a plurality of culturing devices 11 is connected to one sampling device 60. Although the present embodiment has described the cell culturing system 10 in which the culturing unit and a sampling unit are separately provided, the cell culturing system 10 may be a device in which the culturing unit and the sampling unit are integrated (unified).

The culturing device 11 includes a culture medium reservoir 14 storing a culture medium, a flow channel 16 provided between the reactor 12 and the culture medium reservoir 14, a plurality of medical bags 18 connected to the flow channel 16, and a waste liquid unit 20 storing a liquid discharged through the flow channel 16.

A hard tank capable of storing a large amount of culture medium is applied to the culture medium reservoir 14. The flow channel 16 is constituted by multiple tubes 22, and the multiple tubes 22 are respectively connected to the reactor 12, the culture medium reservoir 14, the plurality of medical bags 18, and the waste liquid unit 20.

Examples of the plurality of medical bags 18 include a cell solution bag 18A storing a liquid (cell solution) containing cells, a cleaning solution bag 18B storing a cleaning solution, a stripping solution bag 18C storing a stripping solution, and a collection bag (not illustrated) for collecting cultured cells. The cleaning solution is a liquid used at the time of priming of the reactor 12 and the flow channel 16. Examples of the cleaning solution include a buffer solution such as phosphate buffered salts (PBS) and tris-buffered saline (TBS), and a physiological saline solution. The stripping solution is a liquid for stripping the cells cultured by a culture treatment. As the stripping solution, trypsin or EDTA solution can be applied, for example.

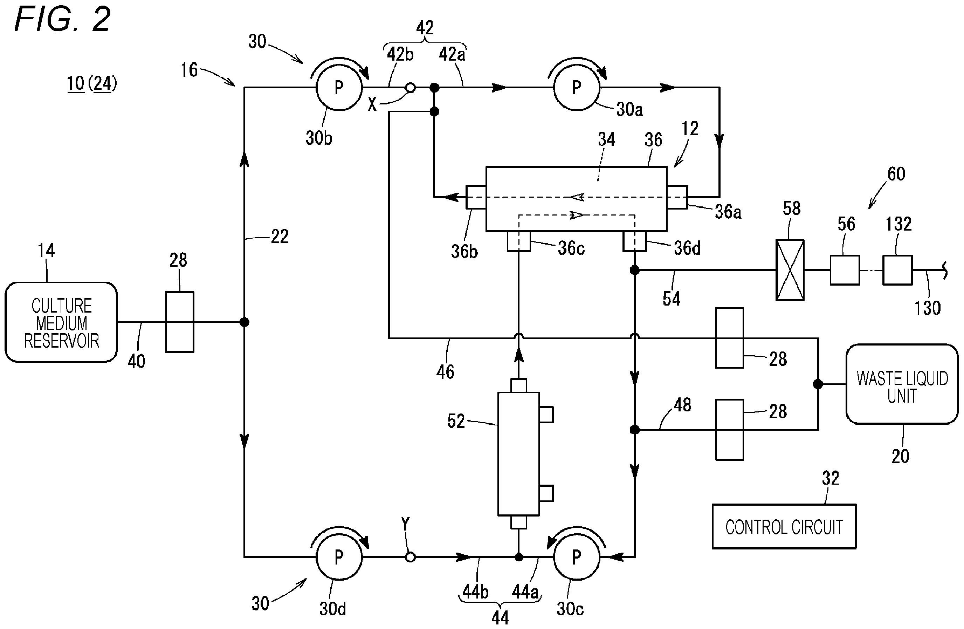

When the cell culturing system 10 is constructed, the flow channel 16 is set so as to pass through a flow path control mechanism 24 of the culturing device 11. The flow path control mechanism 24 includes a housing 26 that houses a part of the flow channel 16. As illustrated in Fig. 2, the flow path control mechanism 24 includes, in the housing 26, a plurality of clamps 28 that opens and closes a predetermined tube 22, a pump 30 that allows a liquid in the tube 22 to flow, and a control circuit 32 that controls operations of the clamps 28 and the pump 30.

The reactor 12 is accommodated in the housing 26 of the flow path control mechanism 24. The reactor 12 includes a plurality of (10,000 or more, for example) hollow fibers 34 and a case 36 that accommodates the plurality of hollow fibers 34. Each of the hollow fibers 34 has a lumen (not illustrated), and cells are seeded on an inner peripheral surface defining the lumen. In addition, each of the hollow fibers 34 has a plurality of pores (not illustrated) that allows communication between the outside and the lumen, and each pore transmits a solution or a low-molecular-weight substance without transmitting cells or proteins. A culture medium or the like is supplied to the cells seeded on the inner peripheral surface of the hollow fiber 34 through the lumen or the pores. Hereinafter, the configuration in which the liquid mainly flows through the lumen of the hollow fiber 34 is also referred to as intra capillary (IC), and the configuration in which the liquid mainly flows through the outer side of the hollow fiber 34 is also referred to as extra capillary (EC).

Each of the cases 36 includes a first IC terminal 36a and a second IC terminal 36b which communicate with the lumens of the hollow fibers 34, and a first EC terminal 36c and a second EC terminal 36d which communicate with a space outside the hollow fibers 34 in the case 36. The tube 22 is connected to each terminal.

As illustrated in Fig. 2, the flow channel 16 includes a medium delivery route 40 connected to the culture medium reservoir 14, and an IC route 42 (internal route) and an EC route 44 (external route) which are branched from the medium delivery route 40. The IC route 42 is a channel for supplying a liquid to the lumen of the hollow fibers 34. The EC route 44 is a path for supplying liquid into the case 36 outside the hollow fibers 34.

The IC route 42 includes an IC circulation circuit 42a capable of circulating liquid with the reactor 12, and an IC supply circuit 42b through which liquid can flow from the culture medium delivery route 40 to the IC circulation circuit 42a. The IC circulation circuit 42a is connected to the first IC terminal 36a and the second IC terminal 36b of the reactor 12, and includes an IC circulation pump 30a that allows liquid to flow through the lumen of the hollow fibers 34. An IC waste liquid circuit 46 that discharges a culture medium to the waste liquid unit 20 is connected to the IC circulation circuit 42a on the downstream side of the reactor 12. On the other hand, the IC supply circuit 42b is provided with an IC supply pump 30b for allowing liquid to flow from the culture medium delivery route 40 to the IC circulation circuit 42a.

On the other hand, the EC route 44 includes an EC circulation circuit 44a capable of circulating liquid with the reactor 12, and an EC supply circuit 44b through which liquid can flow from the culture medium delivery route 40 to the EC circulation circuit 44a. The EC circulation circuit 44a is connected to the first EC terminal 36c and the second EC terminal 36d of the reactor 12, and includes an EC circulation pump 30c that circulates liquid on the outside of the hollow fibers 34. A gas exchanger 52 is provided upstream of the reactor 12 in the EC circulation circuit 44a. The gas exchanger 52 discharges carbon dioxide mixed in the culture medium, and mixes a predetermined gas component (for example, nitrogen N₂: 75%, oxygen O₂: 20%, carbon dioxide CO₂: 5%) with the culture medium. An EC waste liquid circuit 48 that discharges a culture medium to the waste liquid unit 20 is connected to the EC circulation circuit 44a on the downstream side of the reactor 12. The EC supply circuit 44b is provided with an EC supply pump 30d for allowing liquid to flow from the culture medium delivery route 40 to the EC circulation circuit 44a.

Although not illustrated, a plurality of medical bags 18 (cell solution bag 18A, cleaning solution bag 18B, and stripping solution bag 18C) is connected to the IC supply circuit 42b on the upstream side of the IC supply pump 30b or the EC supply circuit 44b on the upstream side of the EC supply pump 30d via a plurality of tubes 22 in addition to the culture medium reservoir 14. Note that these medical bags 18 may be replaced with a collection bag or the like using a sterile connecting device that sterilizes and bonds the bag depending on the intended use.

Then, the sampling device 60 is connected to the EC circulation circuit 44a of the culturing device 11 at a position (between the reactor 12 and the EC waste liquid circuit 48) near the downstream side (second EC terminal 36d) of the reactor 12. Therefore, one end of a sample outflow channel 54 through which a culture medium as a liquid sample flows out is connected to the EC circulation circuit 44a. A culturing-device-side connector 56 is provided at the other end of the sample outflow channel 54. The culturing-device-side connector 56 is mutually connectable to a sampling-device-side connector 132 of the sampling device 60.

An aseptic filter 58 is provided at an intermediate position of the sample outflow channel 54. The aseptic filter 58 maintains an aseptic state of the culture medium circulating through the culturing device 11 (EC circulation circuit 44a). In the sampling device 60, the sample outflow channel 54 may be connected to the IC circulation circuit 42a on the downstream side (second IC terminal 36b) of the reactor 12.

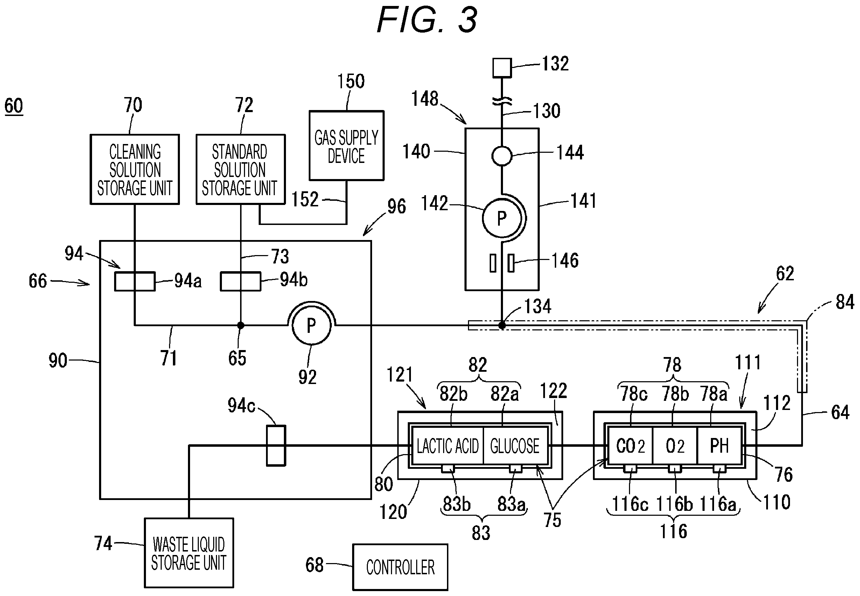

Next, a configuration of the sampling device 60 will be described with reference to Fig. 3. The sampling device 60 collects a sample of a culture medium from one or more culturing devices 11, and detects components contained in the sample and amounts (concentrations) of the components. The sampling device 60 includes a sampling kit 62 having a sampling channel 64 through which a sample is collected, a plurality of mechanisms 66 in which the sampling kit 62 is detachably set, and a controller 68 that controls operations of the plurality of mechanisms 66. The sampling kit 62 is a disposable product that is used and thrown away, and the plurality of mechanisms 66 is a reusable product that can be reused.

In addition to the sampling channel 64, the sampling kit 62 includes a cleaning solution storage unit 70, a standard solution storage unit 72, a waste liquid storage unit 74, and a detection unit 75 (first detection unit 76 and second detection unit 80). The sampling channel 64 is constituted by a flexible tube having an appropriate thickness by which the sample can pass therethrough. The cleaning solution storage unit 70 is connected to a branch point 65 to which one end of the sampling channel 64 is connected via a cleaning solution branch path 71, and the standard solution storage unit 72 is connected to the branch point 65 via a standard solution branch path 73. The other end of the sampling channel 64 is connected to the waste liquid storage unit 74.

The cleaning solution storage unit 70 and the standard solution storage unit 72 are made of, for example, a soft resin material such as polyvinyl chloride or polyolefin, and formed into a bag shape (medical bag). The cleaning solution storage unit 70 and the standard solution storage unit 72 are not particularly limited as long as they can store liquid. The waste liquid storage unit 74 shares a tank of the waste liquid unit 20 of the culturing device 11, but it is not limited thereto, and a medical bag or the like may be applied.

The cleaning solution storage unit 70 stores a cleaning solution. The cleaning solution is not particularly limited, and for example, a buffer solution, a physiological saline solution, or the like described as the cleaning solution in the cleaning solution bag 18B of the culturing device 11 may be appropriately employed.

The standard solution storage unit 72 stores a standard solution. The standard solution is a liquid for calibrating the first detection unit 76 and the second detection unit 80. The standard solution is a liquid whose PH value, glucose value (glucose concentration), and lactic acid value (lactic acid concentration) are set to prescribed values. The standard solution storage unit 72 is connected to a gas supply device 150 (gas supply unit) to be described later, and has an O₂ value (oxygen concentration) and a CO₂ value (carbon dioxide concentration) that are set to prescribed values by the gas supply device 150 supplying O₂ and CO₂ which are predetermined gas components. The sampling device 60 may include two or more standard solution storage units 72 that store standard solutions having different prescribed values, and perform two-point calibration for the first detection unit 76 and the second detection unit 80 by supplying two or more types of standard solutions at different timings.

The first detection unit 76 and the second detection unit 80 are provided in series and separated from each other at an intermediate position of the sampling channel 64. Note that the detection unit 75 is not limited to be divided into the first detection unit 76 and the second detection unit 80, and may have a structure in which the first detection unit 76 and the second detection unit 80 are integrated, or a structure divided into three or more units.

The first detection unit 76 is a tubular member having multiple first elements 78 that come in contact with (in liquid contact with) the sample in a flow path in the sampling channel 64. Examples of the multiple first elements 78 include a PH chip 78a for measuring the PH in the sample, an O₂ chip 78b for measuring the O₂ concentration in the sample, and a CO₂ chip 78c for measuring the CO₂ concentration in the sample. The PH chip 78a is colored by reaction with H⁺ and OH-. The O₂ chip 78b is colored by reaction with O₂. The CO₂ chip 78c is colored by reaction with CO₂.

The second detection unit 80 is a tubular member having multiple second elements 82 that come in contact with (in liquid contact with) the sample in the flow path in the sampling channel 64, and is provided on the downstream side (waste liquid storage unit 74 side) with respect to the first detection unit 76. For example, the multiple second elements 82 are biosensors that react an enzyme with a circulating sample and detect a current change or the like. Examples of the multiple second elements 82 include a glucose chip 82a that measures the glucose concentration in the sample and a lactic acid chip 82b that measures the lactic acid concentration in the sample.

The glucose chip 82a is electrically connected to a glucose terminal 83a protruding to the outside of the tubular member. The lactic acid chip 82b is electrically connected to a lactic acid terminal 83b protruding to the outside of the tubular member. The glucose terminal 83a and the lactic acid terminal 83b are preferably integrated as an electrode terminal 83 with an insulating material therebetween.

In addition, the sampling kit 62 includes a connection part 84 to which one or more sample introduction channels 130 described later can be connected between the branch point 65 of the sampling channel 64 and the first detection unit 76. The connection part 84 is, for example, a member obtained by integrally molding a plurality of branch ports each having a valve (not illustrated) that closes when the sample introduction channel 130 is not attached and opens as the sample introduction channel 130 is attached (in Fig. 3, the connection part 84 is indicated as a range surrounded by a two-dot chain line for convenience). Alternatively, a port to which the sample introduction channel 130 can be connected with the sampling channel 64 being kept aseptic can be applied to the connection part 84.

A part of the sampling kit 62 described above is set in a main mechanism 90 which is one of the plurality of mechanisms 66 as illustrated in Fig. 3. The main mechanism 90 also includes, in the housing 91 (see Fig. 1), a main-mechanism-side pump 92 and a plurality of clamps 94 that opens and closes flow paths in the respective channels (tubes). Although not illustrated, it is preferable that the controller 68 that controls the sampling device 60 is also provided in the main mechanism 90.

The sampling channel 64 extending between the branch point 65 and the connection part 84 is disposed in the main-mechanism-side pump 92. The main-mechanism-side pump 92 has a circular wound portion around which the sampling channel 64 can be wound so as to wrap around, and rotates so as to apply a peristaltic action on the wrapping sampling channel 64 (tube), thereby allowing an internal fluid (liquid, air, etc.) to flow.

The multiple clamps 94 include a cleaning solution clamp 94a for opening and closing the cleaning solution branch path 71, a standard solution clamp 94b for opening and closing the standard solution branch path 73, and a waste liquid clamp 94c for opening and closing the sampling channel 64 between the second detection unit 80 and the waste liquid storage unit 74.

The sampling kit 62 is set in the main mechanism 90, by which a main unit 96 of the sampling device 60 is constructed. The main unit 96 (main mechanism 90) includes a stand 98 for suspending the cleaning solution storage unit 70 and the standard solution storage unit 72 on the top of the housing 91, and has a door-like monitor 100 on the front surface of the housing 91.

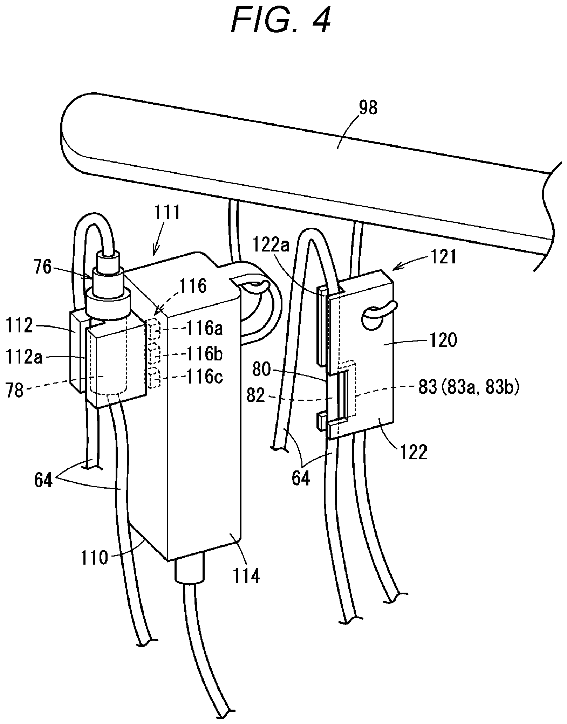

As illustrated in Figs. 3 and 4, the first detection unit 76 of the sampling kit 62 is set in a first measuring instrument 110 which is one of the plurality of mechanisms 66. The first measuring instrument 110 includes a holder 112 having a square tube shape and accommodating the plurality of first elements 78, and a cylindrical measurement body 114 to which the holder 112 is fixed and which optically measures the plurality of first elements 78. The holder 112 is formed to have light shielding properties, and includes a recess 112a for housing and holding the first detection unit 76 from the side.

The measurement body 114 is provided with optical detectors 116 that are arranged to face the plurality of first elements 78 (PH chip 78a, O₂ chip 78b, and CO₂ chip 78c) with the first detection unit 76 being held by the holder 112. That is, the plurality of optical detectors 116 includes a PH detector 116a, an O₂ detector 116b, and a CO₂ detector 116c. Under the control of the controller 68, each optical detector 116 emits measurement light having a wavelength corresponding to the characteristics of the corresponding first element 78, and receives excitation light generated from the first element 78 by excitation. Thus, each optical detector 116 transmits a detection signal based on the degree of coloration of the corresponding first element 78 to the controller 68.

Further, the second detection unit 80 of the sampling kit 62 is set in a second measuring instrument 120 which is one of the plurality of mechanisms 66. The second measuring instrument 120 includes a plate-like case 122 capable of accommodating the plurality of electrode terminals 83 protruding from the plurality of second detection units 80. The case 122 has a recess 122a for housing and holding the second detection units 80 from the side, and a port (not illustrated) into which the electrode terminals 83 are inserted.

The second measuring instrument 120 includes an enzyme detector (not illustrated) electrically connected to the glucose terminal 83a and the lactic acid terminal 83b in a state where the second detection unit 80 is held in the case 122. The enzyme detector detects a current value from each of the glucose chip 82a and the lactic acid chip 82b, and transmits a detection signal based on the current value to the controller 68.

In the sampling device 60, a first sensor unit 111 is constructed by setting the first detection unit 76 in the first measuring instrument 110, and a second sensor unit 121 is constructed by setting the second detection unit 80 in the second measuring instrument 120. The first sensor unit 111 and the second sensor unit 121 are provided to be separated from each other, whereby different detection methods are possible and the respective sensor units can be easily set.

Then, in order to introduce a sample to be measured by the first sensor unit 111 and the second sensor unit 121, the sample introduction channel 130 is connected to the connection part 84 of the sampling kit 62 (sampling channel 64) as illustrated in Fig. 3. Similar to the sampling channel 64, the sample introduction channel 130 is constituted by a flexible tube having an appropriate thickness by which the sample can pass therethrough.

The sample introduction channel 130 has, at one end, a sampling-device-side connector 132 to be connected to the culturing-device-side connector 56 (see also Fig. 2). A plug (not illustrated) attachable to and detachable from the connection part 84 is provided at the other end of the sample introduction channel 130. Hereinafter, a portion where the sample introduction channel 130 is connected to the sampling channel 64 is referred to as a connection point 134.

A part of the sample introduction channel 130 is detachably set to an introduction mechanism 140 which is one of the plurality of mechanisms 66. The introduction mechanism 140 includes, within the housing 141, an introduction-mechanism-side pump 142 (second pump), a pressure sensor 144 that detects the pressure in the flow path of the sample introduction channel 130, and a bubble sensor 146 that detects air bubbles in the flow path of the sample introduction channel 130.

The introduction-mechanism-side pump 142 has a circular wound portion around which the sample introduction channel 130 can be wound so as to wrap around, and rotates so as to apply a peristaltic action on the wrapping sample introduction channel 130 (tube), thereby allowing an internal fluid (liquid, air, etc.) to flow. The introduction mechanism 140 including the introduction-mechanism-side pump 142 is preferably set near the connection point 134.

The sample introduction channel 130 is set in the introduction mechanism 140, by which an introduction unit 148 of the sampling device 60 is constructed. The introduction unit 148 is configured such that a part of the sample introduction channel 130, the introduction-mechanism-side pump 142, the pressure sensor 144, and the bubble sensor 146 can be integrally handled. The sample introduction channel 130 shortly extending from the introduction unit 148 is connected to the connection part 84 on the main unit 96.

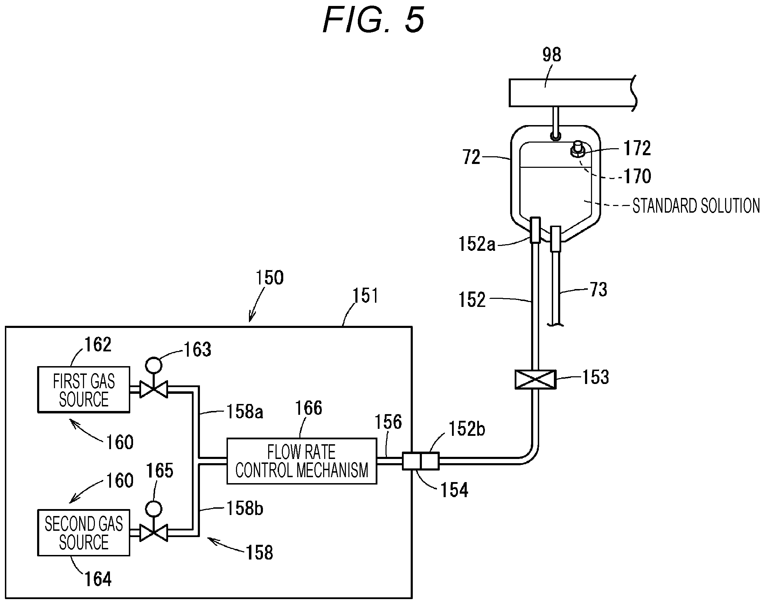

Next, the gas supply device 150 that supplies gas having a predetermined component amount (O₂ concentration, CO₂ concentration, N₂ concentration) to the standard solution storage unit 72 will be described with reference to Fig. 5. The gas supply device 150 bubbles (mixes) gas into the standard solution in the standard solution storage unit 72 to set the gas (O₂ concentration, CO₂ concentration, N₂ concentration) in the standard solution to a prescribed value.

The gas supply device 150 is connected to the standard solution storage unit 72 via a gas supply channel 152 which is a flexible tube. One end of the gas supply channel 152 is connected in advance to one end side of the standard solution storage unit 72 in the longitudinal direction. Specifically, one end 152a of the gas supply channel 152 extends parallel to the standard solution branch path 73 vertically below the standard solution storage unit 72 suspended from the stand 98, and communicates with a space in the standard solution storage unit 72. The one end 152a of the gas supply channel 152 is preferably provided with a check valve (not illustrated) or the like that allows the flow of the gas into the standard solution storage unit 72 and interrupts the flow of the standard solution into the gas supply channel 152.

The other end of the gas supply channel 152 is provided with a gas-supply-channel-side connector 152b that can be attached to and detached from a gas-supply-device-side connector 154 of the gas supply device 150. In addition, the gas supply channel 152 preferably includes an aseptic filter 153 on the gas supply channel 152 in order to sterilize the standard solution.

The gas supply device 150 includes a housing 151 disposed at a position adjacent to the main mechanism 90. The housing 151 includes a common pipe 156 having one end connected to the gas-supply-device-side connector 154 and a plurality of (two in the present embodiment) branch pipes 158 branching from the other end of the common pipe 156. A gas source 160 is connected to each of the plurality of branch pipes 158.

Each of the plurality of gas sources 160 includes a tank or the like that stores a gas having a predetermined component amount in a compressed state. In the following, one of the plurality of gas sources 160 is referred to as a first gas source 162, and the other is referred to as a second gas source 164. The component amount of the first gas of the first gas source 162 and the component amount of the second gas of the second gas source 164 are different from each other. The component amount of the first gas is set such that, for example, the N₂ concentration is 75%, the O₂ concentration is 20%, and the CO₂ concentration is 5%. The component amount of the second gas is set such that, for example, the N₂ concentration is 60%, the O₂ concentration is 30%, and the CO₂ concentration is 10%.

Note that, although the sampling device 60 includes the first gas source 162 and the second gas source 164 in order to perform two-point calibration for calibrating the first sensor unit 111, the gas supply device 150 only needs to include one gas source 160 when one-point calibration is executed. Alternatively, the sampling device 60 may include three or more gas sources 160 in the gas supply device 150 for improving calibration accuracy by the component amounts of three or more different types of gases. It is obvious that the common pipe 156 and the plurality of branch pipes 158 are appropriately provided according to the number of gas sources 160 installed.

An outlet of the first gas source 162 or the branch pipe 158 (hereinafter referred to as a first branch pipe 158a) connected to the first gas source 162 is provided with a first sealing valve 163 that opens and closes a flow path in the first branch pipe 158a. An outlet of the second gas source 164 or the branch pipe 158 (hereinafter referred to as a second branch pipe 158b) connected to the second gas source 164 is provided with a second sealing valve 165 that opens and closes a flow path in the second branch pipe 158b. The first sealing valve 163 and the second sealing valve 165 are opened and closed under the control of the controller 68 to selectively allow the first gas and the second gas to flow out to the common pipe 156.

The common pipe 156 is provided with a flow rate control mechanism 166 for regulating the flow rate of the gas to be supplied to the standard solution storage unit 72. For example, an injector having a valve (not illustrated) whose opening and closing times are controlled is applied as the flow rate control mechanism 166, and the flow rate of the gas is regulated by intermittently opening the valve under the control of the controller 68.

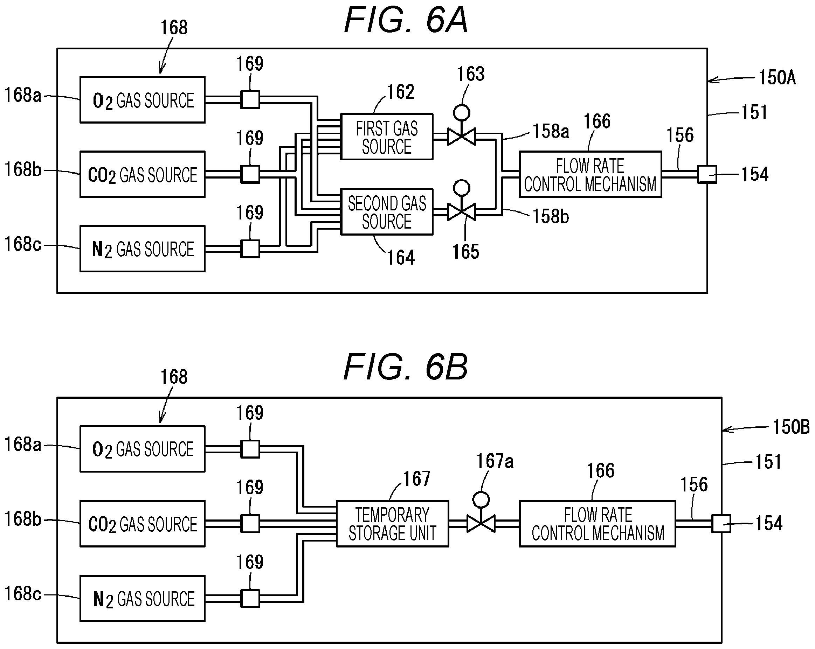

As illustrated in Fig. 6A, a gas supply device 150A may have a configuration in which each of single-component gas sources 168 (O₂ gas source 168a, CO₂ gas source 168b, N₂ gas source 168c) is connected to each of the first gas source 162 and the second gas source 164, and a flow rate control mechanism 169 is provided downstream of each of the single-component gas sources 168. That is, the gas supply device 150A adjusts therein the concentrations of O₂, CO₂, and N₂ to be supplied and generates the first gas of the first gas source 162 and the second gas of the second gas source 164. Alternatively, as illustrated in Fig. 6B, a gas supply device 150B may include one temporary storage unit 167 to which each of the single-component gas sources 168 (O₂ gas source 168a, CO₂ gas source 168b, N₂ gas source 168c) is connected and one sealing valve 167a. That is, the gas supply device 150B generates the first gas from the components in the single-component gas sources 168 in the temporary storage unit 167 and supplies the first gas to the standard solution storage unit 72. Then, the gas supply device 150B generates the second gas in the same temporary storage unit 167 and supplies the second gas to the standard solution storage unit 72.

On the other hand, the standard solution storage unit 72 includes a discharge port 170 through which the gas in the space can be discharged on the vertically upper side. The gas mixed in the standard solution is discharged through the discharge port 170 with the lapse of time, whereby the component amount of the gas in the standard solution varies. The discharge port 170 is preferably provided with a vent mechanism 172 that allows permeation of gas and interrupts permeation of liquid. Thus, the discharge port 170 can prevent the standard solution from flowing out of the standard solution storage unit 72.

The controller 68 (control unit) is a computer including one or more processors (not illustrated), a memory, an input/output interface, and an electronic circuit. The controller 68 controls the entire sampling device 60 by the processor executing the program stored in the memory. At this time, when determining execution of calibration of the first detection unit 76 and the second detection unit 80, the controller 68 performs ganged control of the main unit 96 and the gas supply device 150. The controller 68 may be a control device integrated with the control circuit 32 of the culturing device 11.

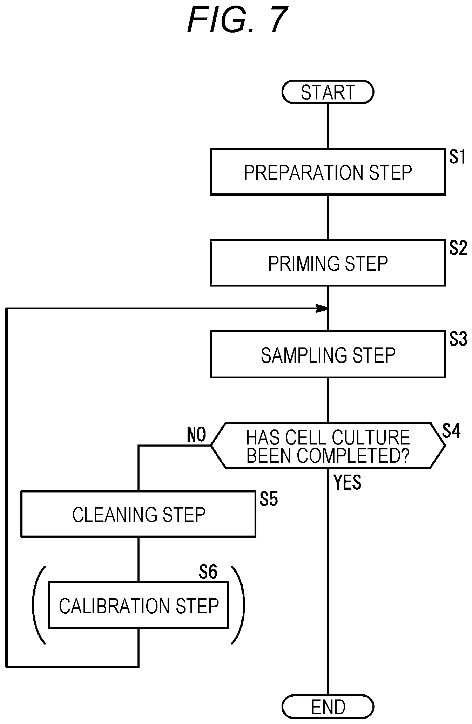

The sampling device 60 according to the present embodiment is basically configured as described above, and a sampling method performed by the sampling device 60 will be described below with reference to Fig. 7. The sampling method includes a preparation step, a priming step, a sampling step, a cleaning step, and a calibration step which are sequentially performed.

First, in the preparation step (step S1), the user of the cell culturing system 10 sets (attaches) the sampling kit 62 to the main mechanism 90 to form the main unit 96 as illustrated in Figs. 3 to 5. Thereafter, the user sets the first detection unit 76 exposed from the housing 91 in the first measuring instrument 110 to construct the first sensor unit 111, and sets the second detection unit 80 which are similarly exposed in the second measuring instrument 120 to construct the second sensor unit 121. The first sensor unit 111 and the second sensor unit 121 are suspended from the stand 98.

Further, the user connects the gas-supply-channel-side connector 152b of the gas supply channel 152 extending from the standard solution storage unit 72 to the gas-supply-device-side connector 154 of the gas supply device 150. The user also sets the sample introduction channel 130 in the introduction mechanism 140 to form the introduction unit 148. Thereafter, the user connects the sampling-device-side connector 132 of the sample introduction channel 130 exposed from the introduction unit 148 to the culturing-device-side connector 56, and connects the plug of the sample introduction channel 130 to the connection part 84.

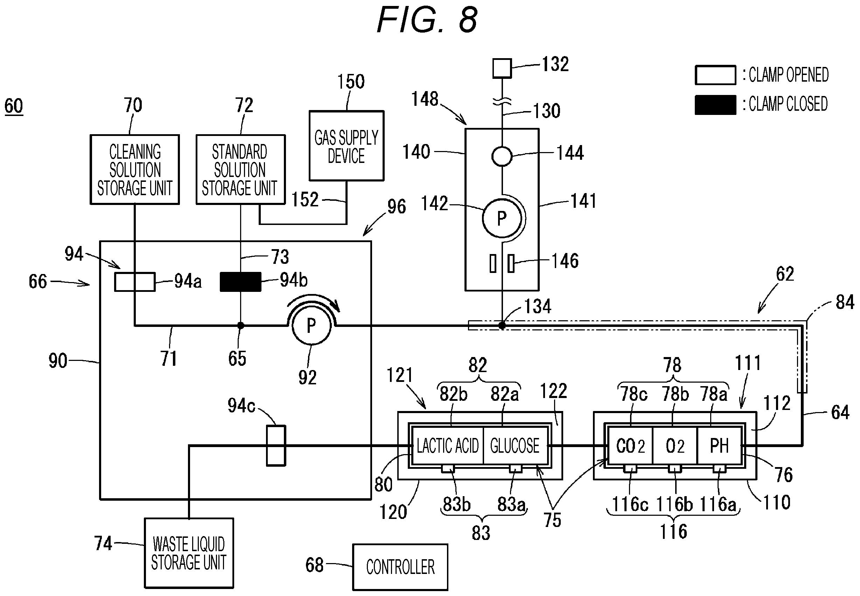

Subsequently, in the priming step (step S2 in Fig. 7), the controller 68 opens the cleaning solution clamp 94a and the waste liquid clamp 94c, closes the standard solution clamp 94b, and rotates the main-mechanism-side pump 92 as illustrated in Fig. 8. Thus, the cleaning solution in the cleaning solution storage unit 70 sequentially flows through the cleaning solution branch path 71, the branch point 65, the main-mechanism-side pump 92, the connection part 84, the first detection unit 76, and the second detection unit 80 and is discharged to the waste liquid storage unit 74.

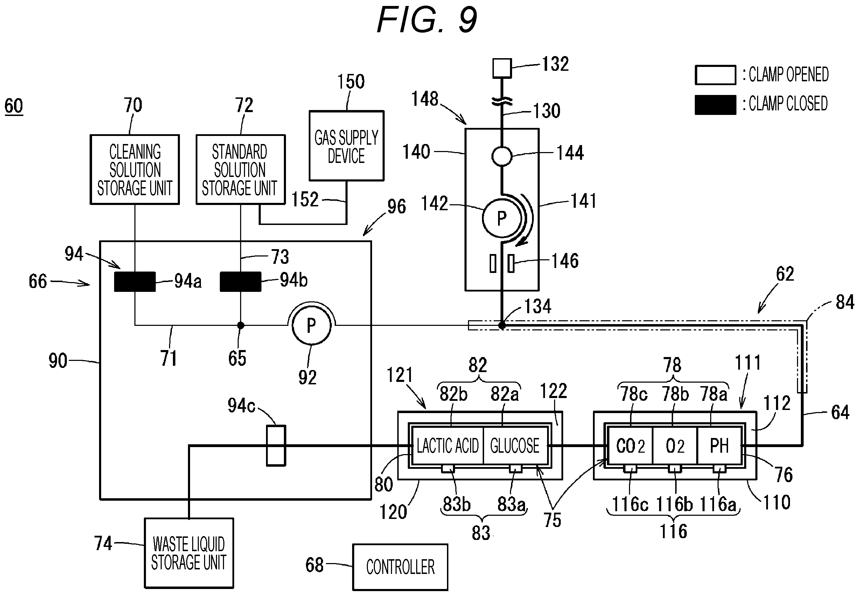

Next, in the sampling step (step S3 in Fig. 7), the controller 68 closes the cleaning solution clamp 94a and the standard solution clamp 94b and opens the waste liquid clamp 94c as illustrated in Fig. 9. The controller 68 also rotates the introduction-mechanism-side pump 142 while stopping the rotation of the main-mechanism-side pump 92. As a result, the sample of the culturing device 11 passes through the aseptic filter 58 (see Fig. 2) and is introduced into the sample introduction channel 130. When flowing through the sample introduction channel 130 and passing through the introduction-mechanism-side pump 142, the sample sequentially flows through the connection part 84 (connection point 134), the first detection unit 76, and the second detection unit 80 and is discharged to the waste liquid storage unit 74.

When the sample passes, the plurality of first elements 78 (PH chip 78a, O₂ chip 78b, and CO₂ chip 78c) of the first detection unit 76 comes into contact with the sample and is colored according to the PH and the contents of O₂ and CO₂. The first measuring instrument 110 optically measures each of the first elements 78, and transmits the detection result to the controller 68. The controller 68 that has received the detection result performs appropriate processing to display the measured values (PH value, O₂ concentration, and CO₂ concentration) on the monitor 100.

Similarly, when the sample passes, the plurality of second elements 82 (glucose chip 82a and lactic acid chip 82b) of the second detection unit 80 comes into contact with the sample, and the second measuring instrument 120 detects current values corresponding to the contents of glucose and lactic acid. The second measuring instrument 120 transmits each detection result to the controller 68. The controller 68 that has received the detection result performs appropriate processing to display the measured values (glucose concentration and lactic acid concentration) on the monitor 100.

After the sampling step, the controller 68 determines whether or not the cell culture of the culturing device 11 is completed (step S4). When the cell culture is not completed (step S4: NO), the cleaning step (step S5) is performed. In the cleaning step, the controller 68 supplies the cleaning solution in the cleaning solution storage unit 70 to the sampling channel 64, as in the priming step illustrated in Fig. 7. As a result, the sample attached to the plurality of first elements 78 (PH chip 78a, O₂ chip 78b, and CO₂ chip 78c) and the plurality of second elements 82 (glucose chip 82a and lactic acid chip 82b) is removed by the cleaning solution.

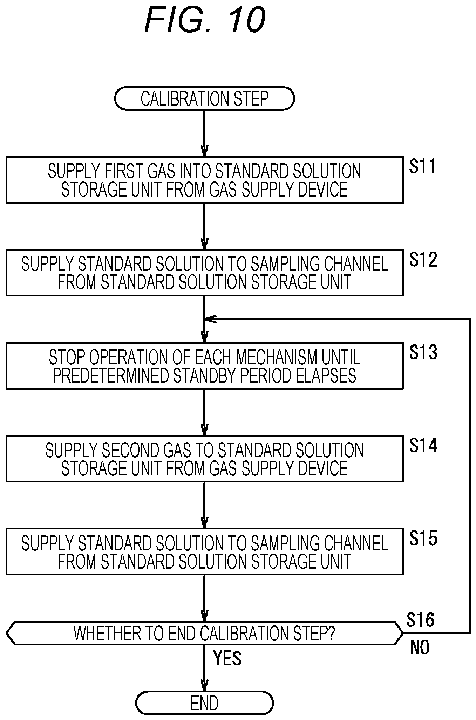

In addition, the sampling device 60 performs a calibration step (step S6 in Fig. 7) as necessary. In the calibration step, the controller 68 performs the calibration step according to the processing flow illustrated in Fig. 10.

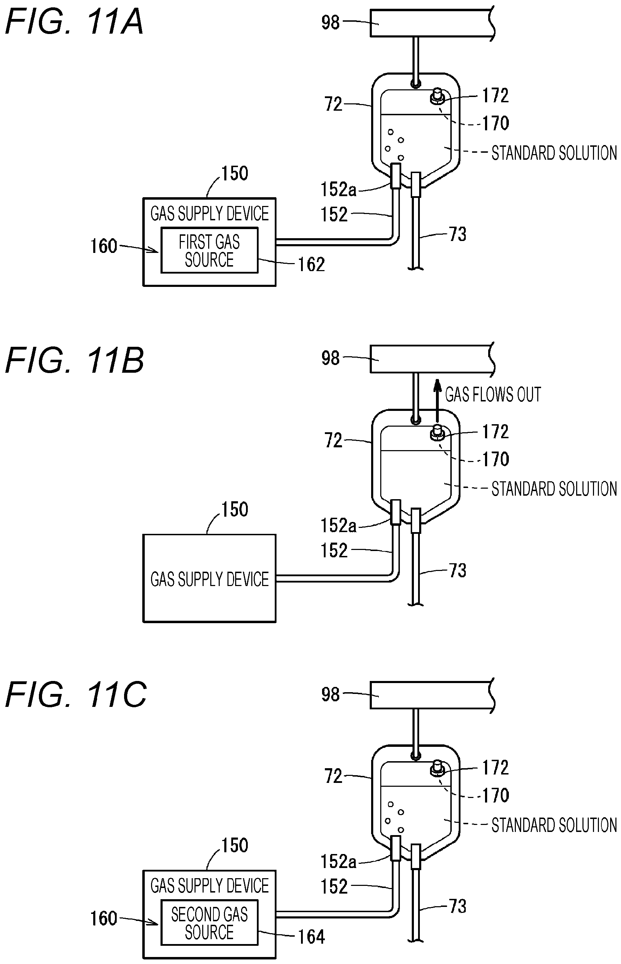

Specifically, first, the first gas is supplied from the gas supply device 150 to the standard solution storage unit 72 (step S11) to change the component amount of gas in the standard solution to the component amount of the first gas. During this process, the controller 68 closes all of the cleaning solution clamp 94a, the standard solution clamp 94b, and the waste liquid clamp 94c, and stops rotation of both the main-mechanism-side pump 92 and the introduction-mechanism-side pump 142. The controller 68 opens the first sealing valve 163 and closes the second sealing valve 165 to allow the first gas from the first gas source 162 to flow out of the gas supply device 150 as illustrated in Fig. 11A. The first gas is supplied to the standard solution storage unit 72 through the gas supply channel 152 with the flow rate of the first gas being regulated by the flow rate control mechanism 166.

The controller 68 continues the supply of the first gas to the standard solution storage unit 72 for a predetermined time so that the standard solution in the standard solution storage unit 72 entirely has the component amount of the first gas. After a lapse of the predetermined time, the controller 68 closes the first sealing valve 163 and stops the operation of the flow rate control mechanism 166.

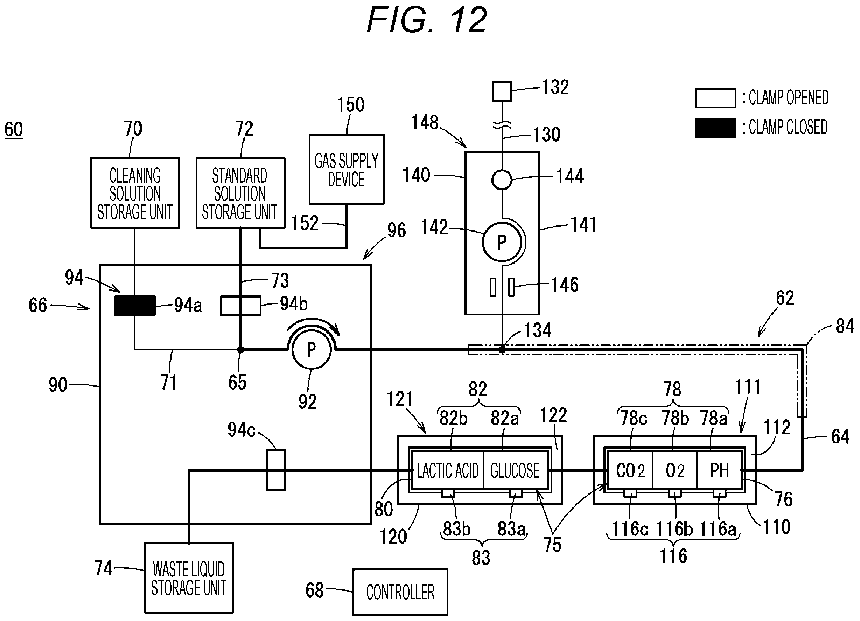

Then, the controller 68 supplies the standard solution adjusted to the component amount of the first gas from the standard solution storage unit 72 to the sampling channel 64 (step S12 in Fig. 10), and calibrates the first measuring instrument 110 and the second measuring instrument 120. Specifically, the controller 68 rotates the main-mechanism-side pump 92 with the standard solution clamp 94b and the waste liquid clamp 94c being opened and the cleaning solution clamp 94a being closed as illustrated in Fig. 12. Thus, the standard solution in the standard solution storage unit 72 sequentially flows through the standard solution branch path 73, the branch point 65, the main-mechanism-side pump 92, the connection part 84, the first detection unit 76, and the second detection unit 80 and is discharged to the waste liquid storage unit 74 due to the action of the main-mechanism-side pump 92.

At this time, the first sensor unit 111 (first measuring instrument 110) measures the PH, the O₂ concentration, and the CO₂ concentration of the standard solution that has been adjusted to the component amount of the first gas, and transmits the measurement results to the controller 68 or the first measuring instrument 110. The controller 68 or the first measuring instrument 110 performs calibration of each of the PH detector 116a, the O₂ detector 116b, and the CO₂ detector 116c (stores a first calibration point) on the basis of the measurement results. The second sensor unit 121 measures the glucose concentration and the lactic acid concentration of the standard solution, and transmits the measurement results to the controller 68 or the second measuring instrument 120. The controller 68 or the second measuring instrument 120 calibrates the second measuring instrument 120 on the basis of the measurement result of the second sensor unit 121.

Furthermore, when step S12 ends, the controller 68 closes the standard solution clamp 94b and the waste liquid clamp 94c, stops the rotation of the main-mechanism-side pump 92, and stops the operation of each mechanism 66 until a predetermined standby period elapses (step S13 in Fig. 10). As a result, the first gas in the standard solution storage unit 72 is discharged to the outside of the standard solution storage unit 72 through the discharge port 170 (vent mechanism 172) during the standby period as illustrated in Fig. 11B.

Then, the controller 68 supplies the second gas from the gas supply device 150 to the standard solution storage unit 72 (step S14 in Fig. 10) to change the component amount of gas in the standard solution to the component amount of the second gas. That is, the controller 68 opens the second sealing valve 165 and closes the first sealing valve 163 to allow the second gas from the second gas source 164 to flow out of the gas supply device 150 (see also Fig. 11C). The second gas is supplied to the standard solution storage unit 72 through the gas supply channel 152 with the flow rate of the second gas being regulated by the flow rate control mechanism 166.

The controller 68 continues the supply of the second gas to the standard solution storage unit 72 for a predetermined time so that the standard solution in the standard solution storage unit 72 entirely has the component amount of the second gas. After a lapse of the predetermined time, the controller 68 closes the second sealing valve 165 and stops the operation of the flow rate control mechanism 166.

Then, the controller 68 supplies the standard solution adjusted to the component amount of the second gas from the standard solution storage unit 72 to the sampling channel 64 (step S15 in Fig. 10), and calibrates the first measuring instrument 110 and the second measuring instrument 120.

The first sensor unit 111 (first measuring instrument 110) measures the PH, the O₂ concentration, and the CO₂ concentration of the standard solution having the component amount of the second gas, and transmits the measurement results to the controller 68 or the first measuring instrument 110. The controller 68 or the first measuring instrument 110 performs calibration of each of the PH detector 116a, the O₂ detector 116b, and the CO₂ detector 116c (stores a second calibration point) on the basis of the measurement results. Then, the controller 68 or the first measuring instrument 110 calibrates the gradient and the height (position) of a calibration curve indicating the relationship between the luminosity and the concentration on the basis of the stored first calibration point and second calibration point. By performing the two-point calibration in this manner, the sampling device 60 can accurately perform the calibration of the first measuring instrument 110.

Finally, the controller 68 determines whether to end the calibration step (step S16 in Fig. 10). For example, in a case where three or more gas sources 160 are provided (step S16: NO), the processing returns to step S13 in Fig. 10, and the processing flow of steps S13 to S15 is repeated. On the other hand, when the supply of the gas from all the gas sources 160 is ended (step S16: YES), the controller 68 ends the calibration step.

Returning to Fig. 7, when the cleaning step (or the calibration step) is completed, the controller 68 returns to step S3 and sequentially performs the subsequent steps. On the other hand, when determining in step S4 that the cell culture is completed (step S4: YES), the controller 68 ends the operation flow of the sampling device 60.

The present invention is not limited to the abovementioned embodiment, and various modifications are possible without departing from the spirit of the invention. For example, the above-mentioned gas supply device 150 is connected to the standard solution storage unit 72 via the gas supply channel 152. However, the sampling device 60 may be configured such that the standard solution storage unit 72 is stored in the gas supply device 150, and the standard solution mixed with the first gas and the second gas in the gas supply device 150 is supplied to the sampling channel 64. Note that the gas supply device 150 may have a configuration in which the tank (first gas source 162 and second gas source 164) is exposed without including the housing 151.

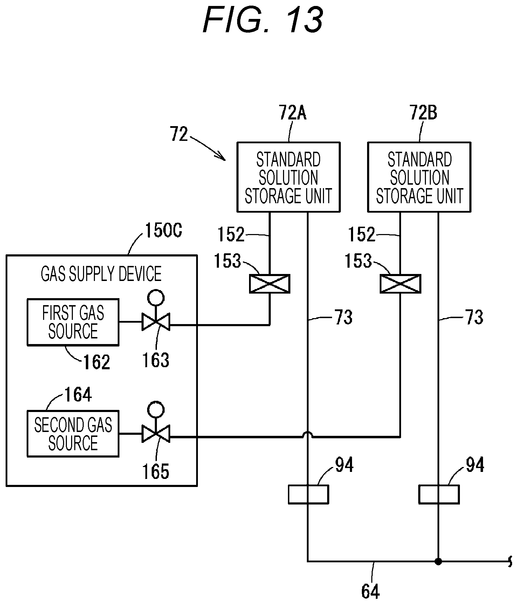

Alternatively, as in a third modification illustrated in Fig. 13, the sampling device 60 may have a plurality of standard solution storage units 72A and 72B, a first gas source 162 of the gas supply device 150C may be connected to the standard solution storage unit 72A, and a second gas source 164 may be connected to the standard solution storage unit 72B. With this configuration, after a standard solution in the standard solution storage unit 72A mixed with the first gas is supplied to the sampling channel 64, a standard solution in the standard solution storage unit 72B mixed with the second gas can be supplied to the sampling channel 64 without setting a standby period. Therefore, the sampling device 60 can quickly perform the calibration step.

In this case, the standard solution in the standard solution storage unit 72A and the standard solution in the standard solution storage unit 72B may have different glucose concentrations and lactic acid concentrations in advance. With this configuration, two different types of standard solutions having different glucose concentrations and lactic acid concentrations are supplied also to the second sensor unit 121 (see Fig. 4), and the second measuring instrument 120 can be subjected to two-point calibration.

Technical ideas and effects that can be grasped from the above embodiment will be described below.

A first aspect of the present invention provides a sampling device 60 for collecting a sample in a liquid form from a culturing device 11 that cultures a cell, the sampling device 60 including: a sampling channel 64 through which the sample flows; a detection unit 75 provided in the sampling channel 64 so as to come into contact with the sample; and a measuring instrument (first measuring instrument 110) that measures at least a gas component contained in the sample by means of the detection unit 75 while the sample flows, the sampling device 60 including a standard solution storage unit 72 capable of supplying a standard solution to the detection unit 75 through the sampling channel 64, and a gas supply unit (gas supply device 150, 150A to 150C) that supplies a gas having a predetermined component amount to the standard solution in the standard solution storage unit 72, wherein the measuring instrument performs calibration by measuring the standard solution mixed with the gas having the predetermined component amount.

With this configuration, the sampling device 60 can easily perform calibration of the measuring instrument (first measuring instrument 110) using the standard solution by supplying the gas having the predetermined component amount from the gas supply unit (gas supply device 150, 150A to 150C) to the standard solution in the standard solution storage unit 72. In addition, the sampling device 60 can eliminate a calibration device that has been used conventionally, whereby it is possible to greatly reduce the burden on the user such as setting the measuring instrument (first measuring instrument 110) in the calibration device.

In addition, the gas supply unit (gas supply device 150, 150A, 150C) includes a plurality of gas sources 160 capable of supplying gases having different component amounts to the standard solution in the standard solution storage unit 72. With this configuration, the sampling device 60 can calibrate the measuring instrument (first measuring instrument 110) at a plurality of points using a plurality of standard solutions to which gases having different component amounts are supplied, whereby the accuracy of calibration can be further improved.

In addition, the gas supply unit (gas supply device 150, 150A, 150C) supplies a first gas from a first gas source 162 that is one of the plurality of gas sources 160 to the standard solution in the standard solution storage unit 72, and supplies a second gas from a second gas source 164 that is one of the plurality of gas sources 160 at a timing different from a supply timing of the first gas. With this configuration, the sampling device 60 can supply the standard solution mixed with the first gas and the standard solution mixed with the second gas to the measuring instrument (first measuring instrument 110) at different timings.

In addition, the gas supply unit (gas supply device 150) supplies the second gas to the standard solution storage unit 72 after a predetermined standby period has elapsed after stopping the supply of the first gas to the standard solution storage unit 72. With this configuration, after using the standard solution mixed with the first gas, the sampling device 60 can supply the second gas to the standard solution in a state where the influence of the first gas is eliminated from the standard solution.

In addition, the gas supply unit (gas supply device 150A, 150B) includes a plurality of single-component gas sources 168 that stores different single gas components, and generates the gas having the predetermined component amount by adjusting a supply amount of the gas component supplied from each of the plurality of single-component gas sources 168. With this configuration, the gas supply unit can efficiently supply a target gas component to the standard solution storage unit 72.

In addition, the sampling device 60 includes: a pump (main-mechanism-side pump 92) that supplies the standard solution from the standard solution storage unit 72 to the detection unit 75; and a control unit (controller 68) that controls operations of the pump and the gas supply unit (gas supply device 150, 150A to 150C), and when calibrating the measuring instrument (first measuring instrument 110), the control unit stops the pump, supplies the gas having the predetermined component amount from the gas supply unit to the standard solution storage unit 72 for a predetermined time, and then operates the pump to supply the standard solution to the detection unit 75. With this configuration, the sampling device 60 can appropriately switch between the supply of the gas to the standard solution and the supply of the standard solution to the detection unit 75, and can stably introduce the standard solution having the gas of the predetermined component amount into the detection unit 75.

In addition, the standard solution storage unit 72 includes, at one end in a longitudinal direction, an outflow channel (standard solution branch path 73) through which the standard solution flows out to the sampling channel 64, and a gas supply channel 152 connecting the standard solution storage unit 72 and the gas supply unit (gas supply device 150, 150A to 150C) is connected to the one end in the longitudinal direction. With this configuration, the sampling device 60 can directly mix the gas into the standard solution accumulated on the lower side in the vertical direction when supplying the gas to the standard solution storage unit 72 held such that one end in the longitudinal direction is positioned on the lower side in the vertical direction.

In addition, the standard solution storage unit 72 has a discharge port 170 through which the gas in the standard solution storage unit 72 is releasable on another end side in the longitudinal direction. With this configuration, the standard solution storage unit 72 can smoothly discharge the internal gas through the discharge port 170.

In addition, the discharge port 170 is provided with a vent mechanism 172 that allows permeation of gas and interrupts permeation of liquid. With this configuration, the standard solution storage unit 72 can prevent leakage of the standard solution to the outside when, for example, it is handled by a user.

A second aspect of the present invention provides a cell culturing system 10 including a culturing unit (culturing device 11) for culturing a cell, the cell culturing system 10 including: a sampling channel 64 through which a sample in a liquid form collected from the culturing unit flows; a detection unit 75 provided in the sampling channel 64 so as to come into contact with the sample; and a measuring instrument (first measuring instrument 110) that measures at least a gas component contained in the sample by means of the detection unit 75 while the sample flows, the cell culturing system 10 including a standard solution storage unit 72 capable of supplying a standard solution to the detection unit 75 through the sampling channel 64, and a gas supply unit (gas supply device 150, 150A to 150C) that supplies a gas having a predetermined component amount to the standard solution in the standard solution storage unit 72, wherein the measuring instrument performs calibration by measuring the standard solution mixed with the gas having the predetermined component amount. With this configuration, the cell culturing system 10 can easily perform calibration of the measuring instrument, and can greatly reduce the burden on the user.

## Claims

1. A sampling device for collecting a sample in a liquid form from a culturing device that cultures a cell, the sampling device comprising:
a sampling channel through which the sample flows;
a detection unit provided in the sampling channel so as to come into contact with the sample; and
a measuring instrument that measures at least a gas component contained in the sample by means of the detection unit while the sample flows,
the sampling device including
a standard solution storage unit capable of supplying a standard solution to the detection unit through the sampling channel, and
a gas supply unit that supplies a gas having a predetermined component amount to the standard solution in the standard solution storage unit, wherein
the measuring instrument performs calibration by measuring the standard solution mixed with the gas having the predetermined component amount.

2. The sampling device according to claim 1, wherein
the gas supply unit includes a plurality of gas sources capable of supplying gases having different component amounts to the standard solution in the standard solution storage unit.

3. The sampling device according to claim 2, wherein
the gas supply unit supplies a first gas from a first gas source that is one of the plurality of gas sources to the standard solution in the standard solution storage unit, and supplies a second gas from a second gas source that is one of the plurality of gas sources at a timing different from a supply timing of the first gas.

4. The sampling device according to claim 3, wherein
the gas supply unit supplies the second gas to the standard solution storage unit after a predetermined standby period has elapsed after the supply of the first gas to the standard solution storage unit is stopped.

5. The sampling device according to claim 1, wherein
the gas supply unit includes a plurality of single-component gas sources that stores different single gas components, and generates the gas having the predetermined component amount by adjusting a supply amount of the gas component supplied from each of the plurality of single-component gas sources.

6. The sampling device according to any one of claims 1 to 5, further comprising:
a pump that supplies the standard solution from the standard solution storage unit to the detection unit; and
a control unit that controls operations of the pump and the gas supply unit, wherein,
when calibrating the measuring instrument, the control unit stops the pump, supplies the gas having the predetermined component amount from the gas supply unit to the standard solution storage unit for a predetermined time, and then operates the pump to supply the standard solution to the detection unit.

7. The sampling device according to any one of claims 1 to 6, wherein
the standard solution storage unit includes, at one end in a longitudinal direction, an outflow channel through which the standard solution flows out to the sampling channel, and
a gas supply channel connecting the standard solution storage unit and the gas supply unit is connected to the one end in the longitudinal direction.

8. The sampling device according to claim 7, wherein
the standard solution storage unit has a discharge port through which a gas in the standard solution storage unit is releasable on another end side in the longitudinal direction.

9. The sampling device according to claim 8, wherein
the discharge port is provided with a vent mechanism that allows permeation of the gas and interrupts permeation of a liquid.

10. A cell culturing system including a culturing unit for culturing a cell, the cell culturing system comprising:
a sampling channel through which a sample in a liquid form collected from the culturing unit flows;
a detection unit provided in the sampling channel so as to come into contact with the sample; and
a measuring instrument that measures at least a gas component contained in the sample by means of the detection unit while the sample flows,
the cell culturing system including
a standard solution storage unit capable of supplying a standard solution to the detection unit through the sampling channel, and
a gas supply unit that supplies a gas having a predetermined component amount to the standard solution in the standard solution storage unit, wherein
the measuring instrument performs calibration by measuring the standard solution mixed with the gas having the predetermined component amount.
